# EUROPEAN PATENT APPLICATION

(11) **EP 4 840 402 A1**
(43) Date of publication of application: **18.11.2026**
(21) Application number: 25176790.1
(22) Date of filing: 15.05.2025
(51) Int. Cl.: A61B 6/06, A61B 6/40, A61B 6/03, A61B 6/46, A61B 6/00, G06T 11/00

(54) **AUTOMATIC ASYMMETRIC SHUTTERING FOR 3D SCANS**

(71) Applicant: Koninklijke Philips N.V., 5656 AE Eindhoven (NL)
(72) Inventor: MINNAARD, Jeffrey, Eindhoven (NL); RUIJTERS, Daniel Simon Anna, Eindhoven (NL); HENDRIKS, Bernardus Hendrikus Wilhelmus, Eindhoven (NL)
(74) Representative: Philips Intellectual Property & Standards

(57) **Abstract**

System (FS) and method for facilitating imaging by a tomographic imaging apparatus having a collimator (COL) capable of asymmetric operation. The system may comprises an input interface (IN) for receiving as input data a specification (s) of a region of interest, ROI, (R) in 3D image domain (*M*) to be imaged for by the tomographic imaging apparatus (IA), the ROI (R) being off-center with respect to an iso-center of the imaging apparatus. A controller (CL) is operable during, or in between, acquisitions by imaging apparatus (IA) of projection frames (*λⱼ*) for tomographic reconstruction into a tomographic image (*m*) of the ROI (R), to cause dynamically adjusting, per frame, an aperture (*A, Aj*) of the collimator (COL) in respect of the so specified ROI.

## Description

### FIELD OF THE INVENTION

The invention relates to a system for imaging, to a related method, to an imaging arrangement including such as system, to a computer program element, and to a computer readable medium.

### BACKGROUND OF THE INVENTION

Cardiovascular afflictions of all manners are still the leading cause for premature death, degradation of quality of life, etc. As per an 11 June 2021 factsheet (available online at https://www.who.int/news-room/fact-sheets/detail/cardiovascular-diseases-(cvds)) by the WHO (World Health Organization), the toll due to CVD (cardiovascular disease) stands at 17.9 million premature deaths worldwide.

Modern medicine provides a range of options, some of which are imaging based. For example, fluoroscopy or angiography may be used that provide live 2D imaging. It allows visualization of anatomic aspects, but also of devices or tools needed in an intervention, such as catheter, stents, etc. Percutaneous coronary interventions (PCI) in a "cath lab" (catheterization laboratory) are one example, where a cardiac stenosis may be treated.

Some projection imaging-based imaging apparatuses ("imager"), such as of the C-arm type, allow obtaining 3D X-ray views in the "cath lab" (catheterization laboratory) that may enable better guidance, e.g. when advancing interventional devices through complex structures in the body. Generating 3D X-ray views in the cath lab is however time consuming, and disrupts user workflow with associated distractions: for example, such 3D imaging requires imager's gantry (e.g., C-arm) be repositioned, well iso-centrically, and distractive attention needs to be paid during such rotational acquisition for this 3D imaging purposes, for example to ensure that there are no obstructions in the rotating gantry's path, etc. In particular, collimation, in general needed to keep radiation dose contributions at bay, can be at times cumbersome to use, in particular when the region of interest to be 3D imaged is off-center the current iso- center of the imaging apparatus.

### SUMMARY OF THE INVENTION

There may therefore be a need for improved X-ray based imaging.

An object of the present invention is achieved by the subject matter of the independent claims where further embodiments are incorporated in the dependent claims. It should be noted that the following described aspect of the invention equally applies to the related method, to the imaging arrangement, to the computer program element, and to the computer readable medium.

According to a first aspect of the invention there is provided a system for facilitating imaging by a tomographic imaging apparatus having a collimator capable of asymmetric operation, comprising: -
- an input interface for receiving as input data a specification of a region of interest, ROI, in 3D image domain to be imaged for by the tomographic imaging apparatus, the ROI being off-center with respect to an (current) iso-center of the imaging apparatus; and
- a controller operable during, or in between, acquisitions by imaging apparatus of projection frames for tomographic reconstruction into a tomographic image of the ROI, to cause dynamically adjusting, per frame, an aperture of the collimator in respect of the so specified ROI.

In embodiments, the collimator adjustment includes repeatedly adjusting at least one blade of collimator so as to define the per-frame aperture.

In embodiments, the collimator is capable of asymmetric blade control.

In embodiments, the controller is so operable based on a set of per frame collimation parameters corresponding to the respective apertures, the set of said per frame collimation parameters determined by a collimation parameter determiner based on the specification.

In embodiments, the iso-center is outside the specified ROI.

In embodiments, the said specification is providable based on a scout image of at least a part of the imaging domain including the ROI.

In embodiments, the system is capable of supporting a user interface, UI, configurable to allow user providing the specification.

In embodiments, the UI is a graphical user interface, GUI.

In embodiments, the ROI comprises two or more disjoint portions.

In embodiments, the said specification is providable by an at least semi-automated specification provider.

In embodiments, the said at least semi-automated specification provider includes a segmentor capable of providing said specification in a segmentation operation.

In embodiments, the at least semi-automated specification provider is based on a trained machine learning, ML, model.

In embodiments, the reconstructed image represents a volume in image domain having a shape of an ellipsoidal cylinder.

In embodiments, the tomographic image (*m*) is reconstructable by a reconstructor capable of applying a tomographic reconstruction algorithm to the acquired projection frames.

In yet another aspect there is provided an imaging arrangement, comprising the system of any one of the preceding claims, and further comprising any one or more of: the imaging apparatus, the UI, the collimator, the specification provider, the reconstructor, a memory on which there is stored at least one of the scout image (*m**), the collimation parameters, and/or the said specification.

In yet another aspect there is provided a computer-implemented method for facilitating tomographic imaging, comprising:-
- receiving as input a specification of a region of interest, ROI in 3D image domain to be imaged for by the tomographic imaging apparatus, the ROI being off-center with respect to an iso-center of the imaging apparatus; and
- during, or in between, acquisitions by imaging apparatus of projection frames for tomographic reconstruction into a tomographic image of the ROI, dynamically adjusting, per frame, an aperture of the collimator in respect of the so specified ROI.

In yet another aspect there is provided a computer program element, which, when being executed by at least one processing unit, is adapted to cause the processing unit to perform the method.

In yet another aspect there is provided at least one computer readable medium having stored thereon the program element.

It has been found herein that asymmetric collimation (the capability of moving collimator blade(s) independently from at least one other such blade) can be harnessed herein for easier, and more rapid 3D imaging even of regions that happen to be off-center to the current iso-center of the imaging apparatus, such as of an interventional imaging apparatus, such as of the C-arm type, or similar. Also, eccentric regions can be imaged easier. The proposed setup may help making collimation more acceptable for medical imaging users, as previous systems could be cumbersome to use. Specifically, with earlier symmetric collimation (shuttering), a clinical relevance may be low to use such collimation in 3D, as one ought to align the region of interest perfectly in the middle of the field of view. Instead, using asymmetric collimation in the proposed manner, the region of interest (ROI) does not necessarily need to be in the center of the field of view (iso-center). Thus, 3D scans can be made with greatly reduced levels of dose radiation, and can be made faster as asymmetric collimation can be done, instead of adjusting other imaging geometry aspect(s), such as position and/or orientation of gantry and/or patient support for centration. Furthermore, the asymmetric collimation allows defining even an eccentric region of interest that cannot be moved to the iso-center because that would lead to collisions within the acquisition trajectory (e.g. with the subject or other).

More specifically, the proposed setup allows efficiently reducing scatter radiation, an important factor that, if not accounted for, may degrade 3D image quality. The amount of scatter is directly related to the size of the volume that is irradiated, and the structures present in this area (e.g., bone and dental implants scatter much more than soft tissue). The X-ray dose that may be needed for a 3D scan can be substantial. Since the ROI is often only a sub-volume of the full cone-beam volume, it would be beneficial to irradiate substantially only the ROI, or to at least reduce exposure of non-ROI portion(s) of anatomy. The ROI however is often not located in the (current) iso-center. What is proposed herein is dynamic, adaptive, asymmetric collimation, in order to make a 3D reconstruction of such sub-volume of interest outside the iso-center, with reduced dose accrued, in line with the "ALARA" principle according to which dose ought to be *"As Low As Reasonably Achievable".*

The proposed solution will be able to reduce the dose by only focusing on the important features and removing all unimportant features. This can be especially helpful in parts of a procedure where it is already known what the medical imaging "focus" is, as in follow-up scan after a first overview scan for example. By asymmetrically collimating, the amount of scatter radiation is reduced since the irradiated volume is smaller and high scatter objects (bone structures, dental implants) that are not clinically relevant can be kept out of the field of view. Therefore, 3D image quality can benefit.

The proposed setup can be used in all manners of tomographic X-ray based imaging, such as cone beam or other, in a manifold of applications, ranging from cardiac to dental and others.

*"user"* relates to a person, such as medical personnel or other, operating the imaging apparatus or overseeing the imaging procedure.

*"subject"* is used herein in the general sense to include animate *"subject"* such as a human or animal patient, or anatomic parts thereof but also includes inanimate subjects such as an item of baggage in security checks or a product in non-destructive testing. However, the proposed system will be discussed herein with main reference to the medical field, so we will be referring to the "subject" as "the patient" or a part of the patient, such as an anatomy or organ, or group of anatomies or organs of the patient.

As to notation used herein, designation "*m* " will be used herein consistently to refer solely to tomographic imagery/image, that is, to the result of a tomographic reconstruction operation from plural multi-directional projection images "*λ* ". Thus, a short-hand reference to *"image(s)*/*imagery m"* is appropriate and will used herein at times. The reconstructed imagery may include a slice or a volume of voxels." *λ*" on the other hand will be used herein consistently as notation for projection imagery. Projection imagery refers in general to plural frames. Projection imagery is a function of imaging geometry g used at time *t* at which the respective frame *(t,g)* is acquired. Projection imagery *λ=λ(t,g)* may be referred herein simply as *"projection imagery*/*images*/*image"* or *"frame(s)" λ* or *λ(t,g).* In order to refer specifically to the time series character or to a given image or frame, notation *λt: = λ(t),mt: =m(t)* may be used at times.

### BRIEF DESCRIPTION OF THE DRAWINGS

Exemplary embodiments of the invention will now be described with reference to the following drawings, which, unless stated otherwise, are not to scale, wherein:-
Fig. 1 is a schematic diagram of an X-ray based tomographic imaging arrangement, in particular of the interventional type as may be used herein in embodiments;
Fig. 2 shows various off-center imaging geometries as may be employed herein;
Fig. 3 is a further illustration of an off-center imaging geometry using asymmetric collimation;
Fig. 4 shows a block diagram of a computer implemented system of facilitating tomographic imaging, in particular with asymmetric collimation reconstruction of off-center region of interest;
Figs 5, 6 show flow charts of methods of facilitating tomographic imaging; and
Fig. 7 shows schematically an imaging geometry with multi-parts region of interest to be reconstructed herein in embodiments.

### DETAILED DESCRIPTION OF EMBODIMENTS

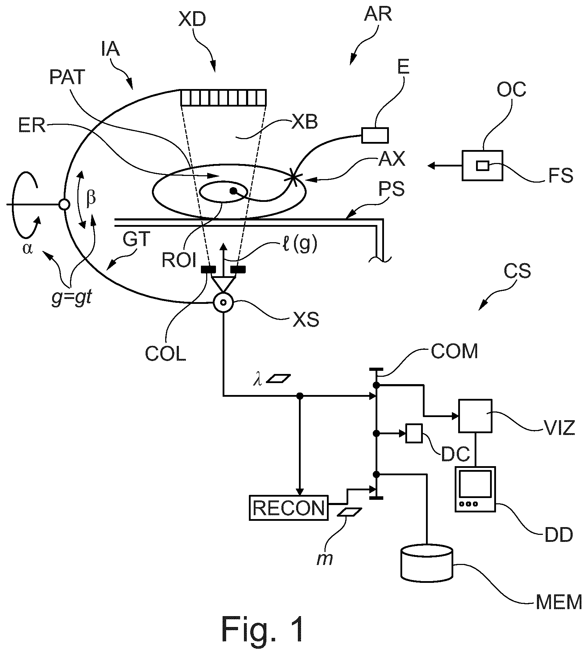

Reference is now made first to the block diagram of Fig. 1 which schematically shows components of an imaging arrangement IAR, particularly the imaging arrangement may be set-up as a medical arrangement that may include the imaging apparatus, preferably of the X-ray type and a computing system the latter type processing the data it receives from the earlier.

Briefly, as will be explained more fully below the imager is capable of multiple imaging modes in some embodiments, in particular in 2D and 3D imaging mode. The computing system may include the functionality FS for efficient 3D imaging, in particular when used in conjunction with asymmetric collimation for better, more rapid 3D imaging if and when needed in addition, or instead, of the 2D imaging. However, the proposed functionality FS is not necessarily confined to such dual mode imaging with 3D, 2D imaging but can also be used instead purely for 3D imaging, without explicit 2D imaging if and when needed.

In embodiments, the imaging arrangement AR may include an interventional X-ray imaging apparatus IA capable of i) allowing an interventionalist (human or robot) access to an imaged subject PAT, and ii) acquiring spatial projection data λ along multiple spatial directions relative to a reference coordinate system. Such imaging apparatus ("imager") may include an imager IA of the C-arm or U-arm type, or similar. Such C-arm type imager IA, as schematically indicated in the Figure, has the eponymous C-shaped gantry GT. Gantry GT forms a cutout, thus affording to medical user such access, and the C-arm GT, which carries imaging components such as X-ray source XS and X-ray detector XD, is movably mounted in order to realize said multi-directional imaging capability. Different spatial field of views (FOVs) can be so realized. However, in alterative embodiments, the imager IA may also be envisaged herein as a (purely diagnostic) CT scanner with doughnut shaped gantry where 3D imaging is the primary concerns, with no displaying of 2D imagery that is only used for reconstruction, as opposed to interventional or other imaging where both, 2D and 3D imaging are for display, the latter if and when needed to complement the 2D mode.

The computing system CS may be data-commutatively coupled, via wireless, wired or hybrid communication infrastructure COM, to imager's detector XD in order to receive therefrom, and process, the spatial data λ acquired by the imaging apparatus. The computing system CS may be partially or wholly integrated into the imaging apparatus, such as in imager IA's operator console OC, or in other computing system, node or device, or at least the computing system may be arranged partly or wholly in the same exam room in which the imager IA is present. In some embodiments, some of the proposed functionalities of the computing system may be arranged remotely, such as 3D reconstruction functionality RECON. Said spatial data as produced by the imaging apparatus IA may be in the form of 2D projection imagery λ. The projection imagery can be visualized by a visualizer VIZ on a display device DD during interventional use of imager IA, for example during user (human or robot) performing a procedure in respect of a region of interest ROI of imaged subject PAT, such as a patient. For example, the region of interest (for the procedure and/or the imaging) may be a lesioned site, such as stenosis in a vasculature of the subject's heart. The procedure may be one of angioplasty to relieve stenosis, but numerous other procedures are envisaged herein.

One or more tool or device E may be introduced through an access point AX into subject PAT, in order to perform or support the procedure. The procedure may proceed over multiple phases, at least one such phase effecting a remedial action in respect of the ROI, such as alleviating the lesion, e.g., broadening the stenosis by placing a stent and/or by operation of balloon catheter, or others.

At least a part of the projection imagery acquired may be provided as a stream of such projection images, referred to as frames herein, that, when displayed/visualized in time sequence, provide a live action video feed. Such feed can be visualized on a display device DD by streaming the projection imagery from detector XD through the communication structure COM to the display device for displaying said video feed, or of still images/frames, as needed. Thus, the imaging arrangement allows user to observe via said live imagery (video or still) internal structures within subject PAT, such as the ROI, intermediate or other anatomical structures R, and the (one or more) tool E itself. In this manner, the imaging arrangement is capable of providing 2D image-guided support to user/procedure.

As mentioned, the procedure may proceed over many phases, such as introduction phase, navigation phase where the device E is navigated towards the ROI through an intricate system of conduits, such as the vasculature. The tool, a catheter, guidewire, etc., may be navigated towards the stenosis in the patient's cardiac vasculature. For example, the user can observe anatomies that are passed allowing the way as the elongated tool E is threaded towards the region of interest, whilst the live action feed of projection imagery is acquired and displayed in real-time on display device DD, thus providing (live-) image-guided support. For example, the user can see on the display real time video that visualizes how the device E is moving, its state is changing, such as its expansion, and also certain physiological activities, such as heart activity or respiratory activities, or other (subsidiary) one or more activity caused by such prime activity.

The set-up of the X-ray imaging apparatus being of the C-arm type or similar, is such that it enjoys a possibly large number of degrees of freedom of adjusting an imaging geometry of the imager so that projection imagery can always be acquired at a most opportune spatial direction ℓ. The spatial direction is a function of the imaging geometry used. The field of view (FOV) is a function of imaging geometry. The imaging geometry, and thus the FOV, may be adjusted potentially many times over during the procedure, depending on imaging context, such as the current region (which may not be the ultimate /actual ROI for the clinical purpose of the procedure), a given portion of an anatomy, phase of procedure, state (expanded/or not) or position, etc., of device E, etc.

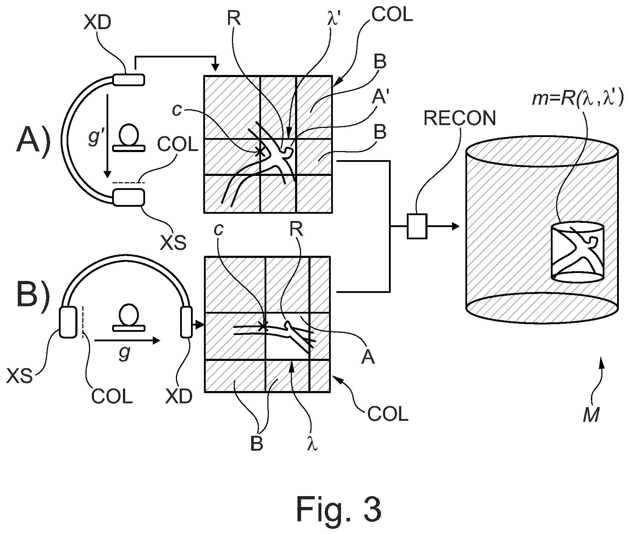

Whilst the imaging apparatus is primarily envisaged herein for 2D imaging, such as the said projection imaging, it may also allow, in addition, in a different mode of operation, 3D imaging, that is, tomographic imaging . In such tomographic imaging, (3D) tomographic imagery *m* (either a single slice, multiple slices, or a volume(s)) may be obtained, for yet better image-guided support of user, or other use, as needed. 2D Projection imaging is still one of the most preferred modes of imaging, due to its simplicity, but of course, it suffers from a certain loss of spatial information in respect of structures that happen to overlap, one or more behind one or more others, along a given projection direction. Their attenuative contributions as recorded by the relevant image pixels at the detector XD are "collapsed", summed as line integrals, into the same image values in projection domain. Tomographic imagery on the other hand allows a 3D view "behind" such structure(s), thereby avoiding or reducing such spatial information loss. Spatial ambiguities are resolved the larger the angular range, ideally 180°, for which projection imagery is available. But even an angular range less than 180° can reveal sufficient spatial insight for a given task at hand, at any rate more such spatial insight than a projection image along a single direction can. As little as two acquisitions at 90° (see for example Fig. 3 below for an illustration) may be sufficient in some cases. Thus, 3D imaging can usefully supplement 2D imaging, for better understanding of functional or structural constitution of the imaged locales, thus can better inform the procedural needs in particular for therapy, but also around aspects of diagnosis and/or planning.

A main purpose for 2D projection imaging as envisaged herein is dynamic real-time image-guided support. The requirements for such 2D projection imaging may change from procedure to procedure, or may vary from phase to phase of the same procedure. In distinction to 2D imaging, the 3D imaging is done herein not primarily in real-time, but supplemental, on demand, alongside the more user activity based 2D imaging. The 2D projection imagery and 3D imagery is primarily used for visual consumption by user. 2D projection imagery and 3D imagery may be displayed concurrently, or one instead of the other. Other data consumption of either the 2D imagery or the 3D imagery, or of both, are not excluded herein, such as processing such imagery for diagnostic or planning purposes or other, rather than for image-guided support as primarily envisaged herein. For example, a data consumer DC, such as an artificial intelligence diagnostics system, may run in parallel to examine the live action feed projection imagery λ and/or the occasional 3D imagery.

What is proposed herein is the above mentioned functionality delivered/implemented by the said computational component FS, either as part of the computing system CS or as a standalone computing system, or jointly delivered by system CS and another computing system. Such computing system CS may be included or may be part of, or may indeed be identical to, the computing system that implements the operator console OC, for example. The functionally and the computing system FS implementing /delivering this may be referred herein by the same reference sign "FS" as the "facilitator (system) FS". The facilitator FS is operable for more convenient, rapidly deployable 3D imaging, thanks to using, in a particular manner, asymmetric collimation as will be explored herein in more detail. The facilitator FS may be realized as a single computational entity or node, or across multiple nodes or entities, such as in a distributed computing setup. Principles of edge computing may be used. The facilitator system FS may be at least partly delivered and/or contracted in as a computing service. Facilitator system FS may be realized in software and/or in hardware, over single or multiple compute nodes.

The asymmetrically operable collimator COL is used to restrict the X-ray beam to the region of interest ROI, thus blocking out unnecessary primary radiation to other parts of the subject PAT, which are not of interest for the current imaging. The proposed system is unlike existing 3D imaging capable set-ups where the region of interest had to be laboriously centered to the iso-center of the rotational 3D apparatus before imaging could commence. Only then could the acquisition of the relevant projection imagery along the acquisition path, such as a circular or other arc around the region of interest center at iso-center commence. The proposed set-up allows doing away with this pre-liminary re-centering, which in the past had included cumbersome aspects of imaging geometry adjustment such as re-positioning of the gantry G ( e.g., of the C-arm), and/or of the patient support PS on which the patient lies or otherwise resides during imaging. Only when such re-positioning was done and the region of interest was indeed at the iso-center, was the acquisition of projection data for 3D imaging purposes run. In the present set-up it is envisaged that the user simply selects suitably the location and or size of the region of interest, and it is in particular the collimator that is reset as will be explained in more detail below. In many cases, such solely collimator-based imaging geometry reset may be sufficient to achieve the desired FOV at a given point in time or at a certain phase of the procedure. However, on occasion an additional reset of gantry and/or patient support may still be done, e.g. for regions very far off. But even so, the need for this to arise can be expected to occur less in the proposed setup, or even then, the other than collimator-based imaging geometry reset can be expected to be small compared to earlier setups. Thus, in the proposed a major part of the imaging reset is collimator based.

In the proposed setup FS, the region of interest R could be anywhere in the imaging domain M or examination region ER, and the imaging can then commence quicker. Instead of re-positioning the gantry or patient support, which consumes more space (equipment and user must move out of the way to avoid collusions) the collimator is operated. Thanks to its asymmetric collimation capability, the beam XB is restricted to the out of center, or more generally, off-center region of interest. On such collimator reset, acquisition or projection imagery of such frames for reconstruction of such off-center 3D region of interest can begin. This allows more rapid, responsive, 3D imaging that can be of benefit herein, in particular, in trauma room settings for example, or in any other situations where rapid 3D imaging support is called for. With the proposed setup, in a large proportion of imaging cases, there is no need for re-centering around iso-center c.

Before explaining in more detail operation of the imaging apparatus, in particular of the facilitator system FS, further imaging context will be provided first on various application scenarios as envisaged herein, and the operation of the imaging apparatus and its components in order to facilitate later in-depth explanations.

The interventional imaging apparatus IA includes the X-ray source XS and the X-ray sensitive detector XD. The X-ray source XS, and opposite thereto, the X-ray sensitive detector XD, are arranged preferably on the rotatable gantry GT. The gantry GT may have a recess in which an examination region ER is defined where the patient PAT, or in particular the region of interest, resides during imaging.

Generally, imaging includes energizing the X-ray source XS, so that an X-ray beam issues forth from a focal spot of the source XS, traverses the examination region ER (with the subject PAT in it) and interacts with patient tissue matter. Interaction of X-ray beam XB with tissue matter causes the beam to be modified. The modified beam may then be detected at the detector XD. DAQ/conversion circuitry (not shown) converts detected intensities into digital imagery, in particular into the projection imagery λ. The patient PAT may reside on a patient support PS such as a table during imaging.

Turning now in more detail to the concept of imaging geometry *g* as envisaged herein in embodiments, such are used in order to realize acquisition of different projection imagery at different FOV views. The imaging geometry *g,* which may change over time *g=gt=g(t)* , may be function of user request via control interface (operator console joystick, or other). The imaging geometry may vary as a function of procedure type, type of interventional device E used, phase of procedure, anatomical variation or peculiarities encountered during the interventional procedure, etc.

Spatial aspects of imaging geometry (with continued reference to Fig. 1) may be represented as pose (orientation and/or position) in 3D space of imaging line *ℓ(g)* in the imaging domain/examination region ER. The imaging domain/examination region ER is the portion of space between the X-ray source XS and the X-ray detector XD, both held by the gantry. The gantry may be C-shaped as in C-arm imager IA. The imaginary imaging line ℓ may run from a center point of imaging surface of the detector XD to a focal spot of source XS. The imaging line ℓ may be related to the general projection direction/view at a given imaging geometry setting. Other spatial aspects may further include shape/width of beam, as may manipulated by setting radiation-opaque blades of optional collimator COL. So setting the collimator blades may form an aperture for the beam to pass through. Other spatial aspects may include the length of line ℓ , which corresponds to the source detector distance (SID) parameter.

The various degrees of freedom (DoF) as may pertain to imaging geometry g, may be parameterized by parameters. Such imaging geometry parameters may pertain to angulation , rotation , shift/translation of any one of gantry GT or patient support PS, and said SID distance. Some of the parameters may parameterize line ℓ in space 3D, and hence at least partly define respective instantaneous FOVt= FOV. Any one or more, in particular all, of the imaging geometry parameters may be adjusted independently by operation of a set of actuators or effectors (not shown), such as server motors, stepper motors, or others, to so effect changes in the imaging geometry and to then acquire, once imaging geometry is reset, respective projection frames in the desired field of view. The imaging geometry, and hence pose of line ℓ, its length etc., may well be changed many times over during the procedure, in particular, as the procedure passes through its different phases. The actuator(s) may act on C-arm GT as it is held in suitable bearings, journalling, etc. in movable arrangement.

The imaging geometry parameters map to different imaging geometry settings. The nature and number of imaging geometry parameters, and related settings are merely exemplary. There may be more or less, or they may be different such parameters/ settings. In general, the parameters for different imaging geometry settings may correspond to different combinations of such parameters. Such parameters may include certain values that pertain to rotation about different axes, and/or lengths, such as one or more shift/translation along one or more respective axis of plural axes. The axes may be of a frame of reference coordinate system. Such imaging geometry settings, and hence parameters combinations, etc., can be set either by the user through said imaging control UI (such as a joystick or other user interface of operating console OC), or may be set automatically by a robot, also envisaged herein. On such setting, the parameters may be translated into lower-level hardware commands. Such hardware command may be propagated via communication infrastructure, such as bus system or other, to respective machine control units of one or more actuators. The one or more actuators may effect the respective imaging geometry re-sets which may cause motion, rotation or both along or about various one or more spatial axes, such as roll, pitch, yaw, etc. Spatially encoding sensors in conjunction with such actuator may be used to track the change in imaging geometry *gt*. Thus, there is an inherent functional dependency of the imaging geometry on time as the procedure unfolds, *gt : =g(t).* The projection imagery *λt* acquired that the different imaging geometries is then itself a function of both, respective imaging geometry and time *t,* λ=λ (*g,t*). And it is such time series *λ=λ (g, t)* which is received as the said live action feed and displayed as video data on the display device DD for user to monitor such device E-based procedure. As said before, displaying of one or more frames as still imagery from among frames *λ=λ (g, t)* is not excluded herein, and some user prefers still imagery over video, at least at certain phases of the procedure. For 3D imaging, of main concern herein, projection imagery *λ(g*,*t)* is acquired over an arc at various imaging geometries *gt* for example, and the sought tomographic image *m* is then reconstructed, based on the acquired projection imagery *λ(g,t)*.

Turning now in more detail to tomographic reconstruction, this is deliverable herein by the said reconstructor RECON. Reconstructor RECON may run a suitable tomographic reconstruction algorithm, such as filtered back-projection (FBP), algebraic, iterative, statistical or machine learning based (such as neural network based, in particular CNN based). Reconstruction as such is a computationally demanding process. The reconstructor functionality RECON may use any one of otherwise known tomographic reconstruction algorithms, in particular, but not necessarily, those tuned to cope with reconstruction of off-center region R. For better responsiveness, the reconstructor RECON can be run on processor circuitry capable of parallel processing, such as on those of a multi-core design. GPUs or any other may be used. Computational operations, such as forward -projections and/or backward-projection operations (on which more below) and data updating, may be formulated along SIMD principles. For example, dot-product or matrix multiplications may be used in order to facilitate any one or more of the said computational operations involved in the reconstruction algorithm. Reconstruction algorithms of the iterative type may be preferred herein. The detector XD comprises an imaging surface. This surface, such as a plane, may be made up of X-ray sensitive pixels arranged in a 2D layout. The detector surface situatable at different positions in space represents the projection domain. In distinction, the portion of 3D space in between source XS and detector XD, within which at least part of the imaged subject resides during imaging, is the image domain *M*. The image domain M is conceptually made of a grid of voxel positions v. Broadly, reconstruction, in particular of the tomographic type, is the computational task of determining slice imagery through the examination regions, and hence through subject. This is implemented by reconstructor RECON. A tomographic volume can be thought of as being made up of plural such slices. In the following, "image *m*" may relate to such a given slice, or the volume. In reconstruction, the voxel positions v are populated with image values, based on the projection data, using back projection and, in iterative reconstruction algorithms, forward projection operations. The projection imagery *λ(g,t)* from which such reconstruction is made is not generally displayed (as opposed to the projection imagery acquired for live action feed display), but such displaying may optionally still be done. The facilitator FS is operable to facilitate such acquisition of projection imagery *λ(g,t)* for the purpose of tomographic reconstruction will now be explained in more detail.

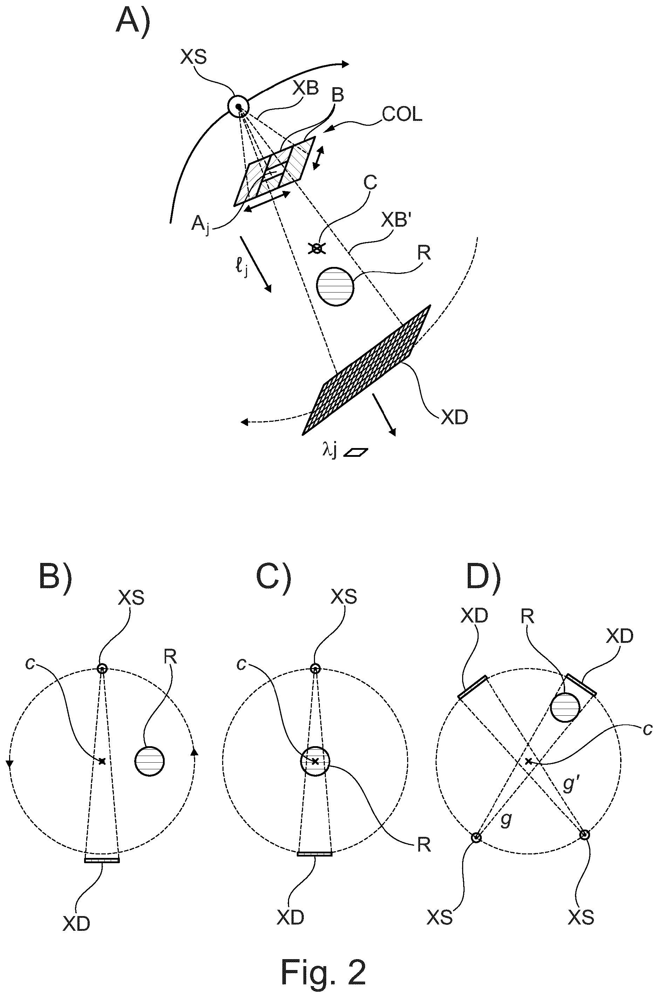

Reference is now made to Figs 2 , which illustrates the above mentioned imaging 3D imaging concepts, in particular for an off-center region of interest R, with asymmetric collimation.

More specific reference is now made first to the perspective view as afforded by Fig. 2A), which illustrates schematically a particular imaging geometry. The imaging geometry there gives rise to imaging line *ℓⱼ* along projection direction j.

The collimator COL includes a set of moveable radio-radiation opaque blade(s) B, that can be independently moved to define a respective imaging aperture Aⱼ, one per direction *j.* The respective aperture A, Aⱼ allows forming X-ray beam XB' out of the native beam XB so conform spatially to the region of interest as the region appears in projection view along any given respective projection direction j as set at any given time.

One or more of the blades B are actuated by suitable actuators, so that the respective forward edges of the blades together delimit an "inter-blade" opening, that is, the aperture A, Aⱼ. It is through this aperture A, Aj that a part of the native radiation beam XB can pass, thereby forming the restricted/collimated beam XB', the form of which is a function of the shape of the aperture Aⱼ.

In a dynamic set-up as envisaged herein, as the rotational geometry changes to new imaging directions *j, j',* the collimator may be re-set to form a respective different apertures Aⱼ, Aⱼ, at which differently collimated respective frames *λ=λj, λj'* are then acquired, and so on, along the acquisition path. As the projection view on the region of interest, which may not necessarily be fully symmetric or may not even be partially symmetric, may change, so does the collimation aperture Aj, in order to, preferably tightly and dynamically, conform to each of the changing projection views encountered during rotation when imager IA is sweeping out the acquisition path. The collimation is tight in the sense that the outer portion of the collimated beam XB' (that is, the portion away from the central imaging line that runs from focal spot FS to center of detector surface) conforms with the outer periphery of the respective projection view on ROI at the current, given, projection direction *j*. However, a small margin may be present nevertheless at times.

In acquisition, acquisition equipment, in this case X-ray source XS and/or X-ray detector XD, are rotated around the region of interest as schematically indicated by the pair of curved and dashed arrows. In addition to such rotation, there is the said asymmetric collimation, based on the current projection direction *j*. Thus, there is in general per frame collimation, or per projection direction *j* collimation.

In addition to such per frame/projection direction j collimation in a dynamic manner, the region of interest R can be off-center (Fig. 2A, B), rather than centered (Fig. 2C). Specifically, Fig. 2B allows a schematic side elevation view taken along the rotational axis of the system, with the rotational axis passing through iso-center c and extending in the plane of the drawing of the Figure. As can be seen, the region of interest R is wholly outside the iso-center c. That is the iso-center c, and thus the rotational axis, does not pass through the region of interest, and this constellation is indeed envisaged herein in some cases, indeed in most cases, for off-center imaging with dynamic asymmetric collimation. In contrast, the elevation view in Fig. 2C) illustrates the case where the region of interest is centered as the iso-center c runs through center of the region of interest R. In general, the term "off-center", as used herein, will indicate that the center of the region of interest does not coincide with the iso-center c. In particular, and more specifically, in some cases the term off-center may imply that the iso-center is not within the region of interest at all. The previously defined term off-center may also be used for regions of interest that are not natively themselves symmetric in any way. For such regions, a nominal center can be defined and the above definition can then be applied to define the term off-center, if the nominal center does not coincide with the iso-center c. The nominal center for such arbitrary shaped regions R may be defined as the (geometric) center of enveloping 3D sphere or other symmetric reference shape, such as a cube or any other that envelopes (circumscribes) the region of interest R.

Fig. 2D) shows the imaging geometry for a reconstruction for off-center region R. It is seen that in such cases, there are projection directions g' where the region, it being off-center, is not in the beam or field of view at all, whilst it is at other imaging geometries g. Thus, for off-center region of interest, the acquisition path is not generally unitary made up of one consecutive continuous path but is instead broken up into distinct sections as can be seen in Fig. 2D). In general, for off-center regions R, the beam passes through the region of interest over an arc which is in general less than 180°, thus leading to a slightly degraded reconstruction. However, even such slightly degraded reconstruction, may still be enough to confirm sufficient 3D information, in any case better than or complimentary to the information confirmed by the displayed 2D imagery. In the proposed set-up, the 2D imagery acquired for the purposes of 3D construction is not in general displayed but can still be done if needed and opportune. For better results in the off-center situation of Fig. 2D, other additional acquisitions at imaging geometry *g',* which are 180° flipped as compared to the imaging geometry g as show in Fig. 2D, may be done. Thus, in such flipped geometries g,g', the positions of detector XD and X-ray source XS are exchanged.

With the proposed system FS, there is no need to take the extra trouble, time and effort to re-adjust other than collimation imaging geometry to effect centration prior to projection data acquisition. Thus, in particular, the C-arm (gantry G) or support PS may not need to be moved. Only the one or more blades of the collimator are adjusted so that beam XB' passes through off center ROI, and is focused there. This (re-)collimation operation can be done faster, and more efficiently, as the bulky gantry may remain as is. There is no need to move personnel or equipment out of the way of the moving gantry, as was the case before when geometry of gantry needed readjusting. Thus, more rapid, less space consuming and distractive imaging is facilitated herein, thanks to the proposed facilitator FS.

Reference is now made to Figs 3A, B, which illustrate different imaging geometries, in this case at 90° angulation *g', g,* with asymmetric collimations. Two different apertures/windows A, A' are illustrated, one per imaging geometry *g, g'*. At least one of the collimator's blades is moved independently from the others, to form collimation windows A, A' off-center of the present iso-center c. Whilst a mere two projection frames at a suitable angular separation, in this case 90°, are sufficient for basic reconstruction, in general the imaging geometry is continuously adapted in an acquisition arc so that acquisition equipment XD, XS sweep out acquisition path, preferably subtending a 180° angular range. In this manner, plural (more than two) different projection frames are at different directions *j,* if need be, with asymmetric collimation. Collimation is preferably done tightly to conform the irradiating portion of beam (leaving aside secondary scatter radiation) to the respective projection view on the ROI for convenient off-center imaging at low dosage cost. Thus, dose can be saved as before with any collimation, but, in addition, because of the asymmetric capability, no re-positioning of the gantry or support is needed for re-centering of off-center region of interest R as illustrated. A collimation reset, which can be effected quickly and non-disruptively, is sufficient. The set of asymmetrically collimated projection data *λ*, *λ* ' is reconstructed by tomographic reconstruction operation *R* into tomographic image *m* = *R(λ, λ').*

Owing to the region of interest R being off-center, the shape of the reconstructed volume *m* may be an elliptic cylinder rather than a circular cylinder. It should be noted that the reconstruction volume with voxels that have contributions from all frames will be an "ellipsoidal cylinder" with an asymmetric cone top and bottom. Also, voxels outside this ideal reconstruction volume can be reconstructed (extended field of view), but the further the voxel is away from the ideal volume, the fewer frames contribute, and the reconstruction will be poorer.

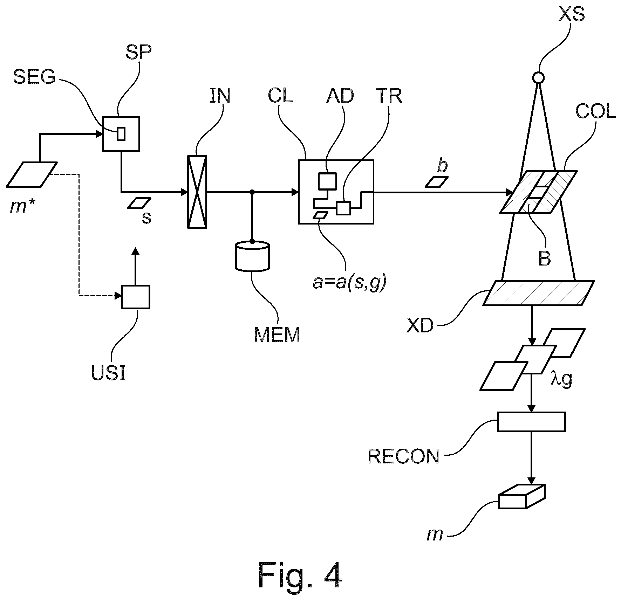

Reference is now made to Fig. 4, which shows a block diagram of imaging facilitator FS preferably configured for off-center 3D imaging with asymmetric collimation. As mentioned earlier, the various components and functionalities of the facilitator system FS may be distributed across different computational nodes such as some one or more being situated at the operation console OC and/or others at a workstation or others more remotely located computational one or more nodes. Alternatively, the whole of the facilitator FS functions are implemented by a single node, such as the console OC or associated workstation, or other computing node.

In general, the facilitator FS may be operable in a pre-programmed mode to compute collimation parameters which can then be used later and applied on the fly dynamically at acquisition. Thus, the operation of the facilitator may be done pre-acquisition in a preparatory phase, if needed. Alternatively, computation and application of collimation parameters are done on-the-fly. Optionally, such computing may be suitably accelerated for example by using one or more GPU's or other multi-core chip sets, with parallel computing, or other.

In operation, at one or more input interface IN, input data is received. Broadly, such input data pertains to spatial specification of the possibly off-center ROI. Based on such specification s, control logic CL processes collimator settings data *a.* The collimator settings data *a= a(s,g)* determine, per imaging geometry *g* to be used (e.g., intended projection direction *j*), the respective aperture for the view along *j* on ROI *R* as per its spatial specification s. In imaging, the settings *a* are applied to collimator COL for asymmetric collimation of possibly off-centered ROI. The settings are applied by issuing from control logic, based on settings *a,* suitable hardware control signals *b* to reset collimator COL's one or more blades B. Collimated projection data λ_{g} is thus acquired at different imaging geometries *g.* So acquired projection data λ_{g} is reconstructed by tomographic reconstructor RECON into 3D tomographic imagery *m* of possibly off-center ROI, thus providing user with on demand 3D insight.

Operation of facilitator FS is now described in more detail. Turning now first in more detail to the input data, this may include in particular 3D specification of the region of interest *R* to be imaged. As said, this region of interest, and hence the specification, may pertain to a portion of image domain that is off-center to the current iso-center *c* of the imaging apparatus to be used. The facilitator may be used in a dynamic image guided procedure, which may use 2D imaging and, occasionally, if needed, the 3D tomographic imaging as facilitated herein.

The specification itself can be formed in manifold ways, all contemplated herein in different embodiments. The ROI specification s may be made up of a sub-set in terms of voxels, their coordinates, etc. It can be defined as a geometric locus, by means of a geometric formula, level surfaces or any other way. In particular, the specification pertains to a 3D portion in 3D space, so is not, in general, a mere specification of a set in 2D. Thus, whilst the ROI is illustrated herein in 2D, such views along viewing direction are for ease of representation only.

Thus, the specification *s* may specify the ROI spatially, such as in terms of any one or more of orientation, location, and extent. The specification of the region of interest may be processed by the facilitator FS into collimation parameters for use in dynamic asymmetric collimation during acquisition of projection data of the ROI, for tomographic reconstruction of the ROI based on such projection data.

The specification may be defined in multiple ways, such as by operation of an at least semi-automated specification provider SP. For example, the specification provider SP may cause a segmentation for the ROI of initial data *m**, such as of an anatomical atlas or a prior image of the subject to be imaged, such as low dose scout scan or any other (image or image based) data. The ROI specifier SP may effect such segmentation by using a segmentor component ("segmentor") SEG. The segmentor SEG may use any image segmentation algorithm, preferably capable of segmentation in 3D. The segmentation algorithm may be used based on machine learning or may be based on any analytic/classically configured segmentation algorithm. Segmentor SEG may run on any suitable computational node with sufficient computational resources. The specifier may cause such segmentor SEG functionality to be contracted in as a, possibly external, computing service. In embodiments, the ROI specification may be done automatically, in particular fully automatically. In another embodiment, the segmentation may be semi-automated in that it is driven or controlled by user input. Thus, in this embodiment, specifier SP may respond to user input. The user input may provide seed data or regularized data to run the segmentation SEG from. For example, in some embodiments, it is the user using a user interface USI where such seed or other control data is provided. The user interface USI may be a graphical user interface ("GUI"). It may support an interactive graphical/visual representation of the image domain. Using such representation, user may use a pointer (input) tool as may be supported by the user interface USI. Such pointer tool may include a computer mouse or touch screen action. The user thus provides geometrical shape, location or other spatial data to define the ROI, such as by circumscribing the region of interest. In some cases, it may be sufficient to merely indicate a single point or multiple point as seed points and this is then auto-expanded in a nominal region, such as a sphere or ellipsoid, a cube or any other. Such nominal region is than thought to define, such as include, outline etc., the medically intended region of interest on which more 3D details are sought. This region is then reconstructed, once asymmetrically collimated projection data is collected. The so user designated portion of image domain may well be off-center to current iso-center, as earlier described. The user can focus solely on the ROI definition and need not mind that this is off-center. Indeed, with the proposed facilitator system FS, user may be oblivious about ROI's off-centeredness.

If the specification s is done by segmentation, this may be a 3D segmentation. If the specification s is done by segmentation, this may natively yield an asymmetric, irregularly shaped set.. However, a regularly symmetric shaped nominal reference region such as sphere, can be still construed as mentioned earlier. Such nominal region may circumscribe the segmented ROI. For example, an enveloping 2D surface in 3D space can be constructed to envelope the segmented region of interest R. The nominal center of such nominal region is so designated, in order to better define the region and/or for easier collimation. Having said that, in the proposed setup no such enveloping or otherwise circumscribing of ROI by such nominal region is necessarily needed as the proposed setup is capable of dealing with irregularly shaped, asymmetric ROI specifications as provided by segmentor SEG. However, such circumscribing by regular symmetric nominal region may still be used, either in actuality or merely conceptually, in order to define whether or not the specified ROI is off-center.

In other embodiments, the specifier SP is wholly driven by user input. Thus, the user may use pointer tool to effectively draw into the image domain representation the outline of the ROI whose reconstruction is sought. Thus, in such embodiments the specifier SP may be a drawing tool, such as CAD kit or other, optionally with a freehand drawing user input interface. Having said that, some automated guidance may still be provided by specifier SP, such as by automated straightening-out or otherwise regularizing user provided outlines (curves) into lines, regularly curved segments, etc., such as by graphical interpolation. Bezier techniques or others may be used.

As to the collimator COL, in some embodiments, this may include as little as two independently and oppositely moveable X-radiation opaque blades (such as made from lead, or other). One blade may be moveable, whereas the other may be stationary, or both (all) are movable. Such collimations are hence capable of defining prism shaped beams. For tight collimation, it may be thus beneficial to define the circumscribing enveloping reference region for the given ROI segmentation, as a projection direction *j* dependent polygon thus matching shape of an intersection formed by the beam and a plane perpendicular to projection direction *j.* In general, the circumscribing enveloping reference region for the given ROI may be based on size and/shape of the projection footprint of the reference region at a given imaging geometry, such as along intended projection direction *j.* And as such projection footprint may change with projection direction *j,* so may the reference region, or the segmented ROI more generally.

Thus, in general, the reference region that may be used to enclose and properly define the region of interest may be a function of the collimator's geometry, in particular of the geometric shape that the collimator is capable of forming. More complex collimators capable of conforming to more complex shapes, not necessarily confined to geometric primitives, such as circular or polygonal, may include more than two blades, such as three, four, or an even larger number. For example, collimator equipment COL envisaged herein may include a leaf collimator previously only used for radiation therapy delivery in linacs etc., but envisaged herein as mounted instead for present collimation needs in the X-ray imager IA. In general, such multi blade/("leaf") collimators allow defining complex, in particular, irregularly shaped regions of interest. The spatial specification s of the ROI in terms of any one or more of shape (regular or irregular), size and location, may be saved in a memory MEM which may be volatile or non-volatile.

Turning now in more detail to the control logic CL, this is operable to process the specification s. Specifically, control logic may compute, based on the specification, an aperture setting *a=aⱼ,* which can be translated into a collimator control signal *b=bⱼ* that can be used to control dynamically the collimator for frame adaption of its aperture. Thus, collimator aperture setting *aⱼ* is adapted based on specification and a given projection direction *j,* so that aperture *Aj* conforms (preferably tightly) to the projection footprint of the ROI specification at given projection direction *j.* As projection direction *j* is varied in suitable (angular) steps, a respective collimator aperture setting *aⱼ* is thus provided. To this end, controller CL may thus include, or may capable of interfacing with, an aperture determiner AD that processes, as just described, the input data, in particular the spatial specification s of region of interest R, into such collimation setting(s)/parameter(s) *aⱼ* which may differ based on the imaging geometry *g* to be used for a given frame, in particular for a given projection direction *j* at which the frame is to be acquired. Thus, the input data may comprise the spatial definition of the region of interest *s,* and, as a second set of data, the respective imaging geometry setting g (e.g., projection direction *j*) to be used per frame. Thus, the aperture determiner AD is operable to receive as input the specification s and the associated imaging geometric setting g for a given frame, and compute based on such data *(s,g)* an associated collimator parameter *a=a(g): =a_{g}* that defines the shape and/or size of the collimator aperture. Such collimator parameter *a_{g}* may pertain to spatial dimensions in one or more directions of a distance between the forward edges of the blades. Thus, the aperture determiner AD may provide a list of such parameters, with one or more parameters per each intended projection direction *j*.

The collimation parameters *a_{g}* (e.g. *aⱼ)* may be processed by translator component TR ("translator"). Specifically, translator TR translates geometric information as encoded in the parameters *a_{g}* into suitable per-frame hardware commands (or signals *b*_{g}), which can be used to control accordingly the blades of the collimator. For example, the parameters *a_{g}* may define a distance in space between opposing blades, and this distance can then be translated to forward or backward motion of the corresponding blades by using spatial encoders in the actuators that spatially actuate the blades into position/orientation.

The collimation parameters *a_{g}* may be made of length of angular specifications for blade settings. Such collimation parameters can be computed by analytic geometry or CAD packages, based on intended imaging and collimator geometry. For example, given the imaging geometry, such as the position of the X-ray source and/or detector relative the current iso-center, and possible other of the any of the above mentioned imaging geometry parameters, intercept theorems (Thales' theorems) or other trigonometric functions and relations, may be used to compute, for each projection view *j*, the necessary distance at which the forward edges of the blade needs to be clear from the imaging line ℓ that runs from the focal spot down to the detector through the iso-center.

Specifically, said distance of the collimator blades from the central imaging line per projection direction *j,* can be obtained analytic-geometrically by running imaginary peripheral geometric rays from the focal spot down to the detector plane, so as to circumscribe, in a plane, the outer perimeter of the projection footprint of the region of interest in that view *j*. The peripheral rays may be intersected with the plane in which the collimator blades run to determine the distance at which the respective forward edges of the blades need to advance or recede relative to the central line.

Alternatively, or in addition, forward- and/or back-projection operation may be used to determine the requisite settings *a_{g}.* The forward projection, which may formally be written as *Πg(R),* pertains to projection information in relation to the ROI R detectable at the detector. The forward projected ROI R forms projection data. Forward projection is a function of imaging geometry g. Forward projection is a mapping operation *Π* going from image domain *M* to projection domain *Λ, Π* : *M -> Λ* ⊃ *Πg(R).* Voxels *v* ∈ *M* making up the, possibly off-center, region of interest R, are mapped into its projection footprint *Πg(R)* as may be detectable at detector, given imaging geometry g. The reverse of *Π* forward operation is back projection operation *B: Λ* ->M. Unlike the forward projection *Π,* which is a many-to-one mapping, the back projection B is a one-to-many mapping. This operation results in a subset in image domain, Π_{g}⁻¹ (λ) ⊂ *M.* The result of back projection can be conceptualized as a "cone" (in the general sense) in respect of projection data on the detector XD's surface. This cone includes all voxels in *M* that are projectable into given forward projection *Πg(R),* given the imaging geometry *g,* and hence in particular projection direction and width of beam. The back-projected cone Π_{g}⁻¹ (λ) may be understood as the pre-image (in the algebraic or set theoretical sense) of the forward projection operation *Π*. Thus, given the ROI specification s in image domain, the projection footprint *Π(s)* can be computed, given imaging geometry parameters. Such footprint can be computed by suing said forward projection operation *Π.* Once the forward footprint is gotten, this can then be back-projected using the back projection operator. The required collimation may then be obtained as the intersection in image domain of the collimation plane and the back-projection cone as provided by the back-projection operation *Π⁻¹.*

Once the parameters *a_{g}* are computed whichever way, these can then be stored in memory and retrieved when needed, in particular when the imaging acquisition is to commence as illustrated on the righthand side of Fig. 4: the parameters, on imaging or in a pre-imaging step, are translated into collimator control commands b which are then applied to the collimator during rotational operation. In such an operation, the actuator(s) are actuated to put the blades into position for each viewing direction j to properly (e.g., tightly) collimate around each of the possibly different projection views of the ROI. As indicated on the righthand side of Fig. 4, once the collimated projection frames at different geometries *g* are acquired, these can be passed on to the reconstructor RECON for reconstruction, based on the projection frames, into the sought tomographic image *m.* The tomographic image *m* may then be displayed, otherwise consumed, or stored, as needed.

As an alternative to the above-described pre-computing mode with subsequent application of the collimation commands *b* in imaging, the described operations of any one or both of the aperture determiner AD and translator TR may be done instead on-the fly during ongoing imaging, as needed, and given the capability of the available computing hardware.

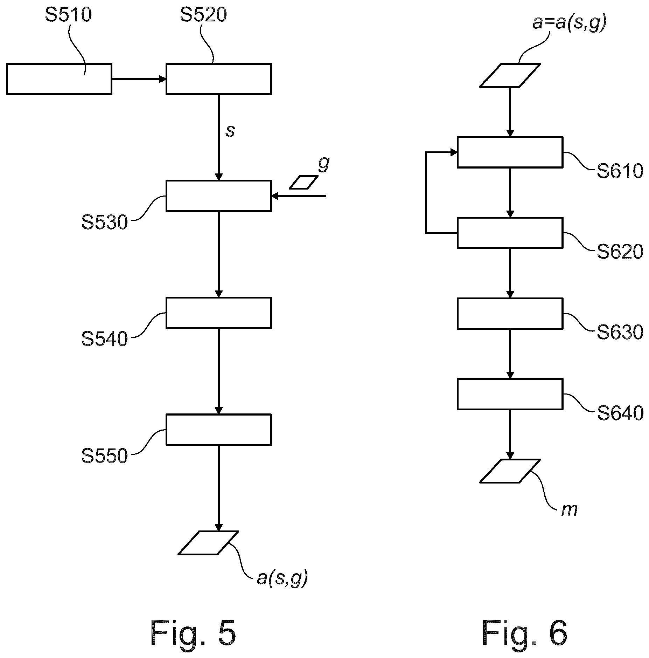

Reference is now made to Figs 5, 6, which show flow charts of a computer implemented method of facilitating tomographic imaging as may be employed herein. The method is not necessarily confined to the architectures discussed above, but may be understood instead as a teaching in its own right.

At step S520, a spatial, preferably in 3D, specification s of the region of interest to be imaged is received. This region, and the specification to which it pertains, may be off-center from the current or expected iso-center of the imaging apparatus to be used for imaging the ROI.

This image domain specification s may be based on a prior reference image *m**, previously acquired (with the same, or with a different imaging apparatus) at step S510. This reference image *m** may be a pre-operative CT volume, an MRI volume, or may be of any other modality, which can be suitably registered for the present purposes onto the imaging domain of the current X-ray imaging apparatus IA. The prior reference image *m** may have been acquired by the same imaging apparatus in a preparatory step, thus yielding, conveniently, native registration.

Segmentation may be used at step S520 to compute such specification. In addition, or instead of such fully automatic segmentation, partial or semi-automatic segmentation may be used, based on user input provided via user interface. For example, user may use such user interface to plant one or more seed points in a representation of the image domain. Such seed points may then be expanded into the desired segmentation. Expansion into standard primitive shapes, such spheres, ellipsoids or cubes, etc. may be used but this is optional as native segmentation of any (irregular) shape is also envisaged herein. The segmentation is in 3D, and thus defines a 3D set of voxels that make up the ROI in image domain. The specification is spatial as it defines any one or more (such as all) of location, shape and size of in-image-domain representation of ROI. The ROI may be off-center.

The segmentation may be machine learning-based, or classical analytic methods may be used instead. In addition, purely manual designation or delineation of the desired, possibly off-center, region of interest is also contemplated. In such embodiments, user uses for example a pointer tool such as a mouse, stylus or touch screen action to define ("draw") the region of interest. If the region is irregular shaped, optionally, this can be standardized by enveloping same into a standard symmetry/regular reference shape to form a convex hull of the possibly irregular shaped region of interest.

The specification s of the region of interest, whichever way obtained, is then received at step S530. As additional input data, a specification of a given imaging geometry to be used is received, in particular including the intended projection directions *j.*

Based on the so specified off-center region of interest R to be reconstructed and imaging geometry *j* to be used, at step S540 the collimation setting/parameter(s) per projection view *j,* and hence per frame, is computed. The collimation setting/parameter(s) *a=aⱼ* may relate to blade positions/orientation and/or to other spatial aspects of the collimator of the imaging apparatus IA. The collimation setting/parameter(s) may be based on respective projection footprint (view) on the specified region of interest, with at least one such projection view per projection direction *j.* Forward projection based method and/or other geometric method may be used in step S540, as for example explained above in connection with Fig. 4

At step S550 the so produced collimation setting/parameter(s) *aⱼ* is/are then made available. Thus, the collimation setting/parameter(s) *aⱼ* may be stored in memory MEM or may be passed on for further processing as is now explained with reference to Fig. 6.

At step S610, the collimation setting/parameter(s) *a= aⱼ are* received.

At step S620, the collimation blades of the collimator of imaging apparatus to be used are adjusted, based on the collimation setting/parameter(s) *a= aⱼ* received at step S610.

At step S630, the collimation setting/parameter(s) *a= aⱼ* are applied to effect collimation during projection frame acquisition (imaging) from multiple directions *j.* This operation S630 results in a set of accordingly, possibly asymmetrically, collimated projection frames, with at least one per view, and acquired at a respective collimation setting. Because of the asymmetric collimation and the off-centeredness of ROI, primary radiation beam may not pass through the current iso-center c in the acquisition, or only a peripheral portion of the beam may pass therethrough.

At step S640, based on the so acquired projection imagery, a tomographic target image *m* is reconstructed. Such step S640 may use a reconstruction algorithm that is preferably configured for off-center reconstruction. In general, such reconstruction algorithm does not introduce error, or at least such error is within a predefined, accepted, margin, when a vector pointing from source XS to respective pixel positions on imaging surface of detector XD is not within a plane defined by a trajectory of source XS. One such reconstruction algorithm is described by H. Schomberg in "A Fourier-filtered backprojection algorithm for cone-beam CT", published in [Proc. Eighth International Meeting on Fully Three-Dimensional Image Reconstruction in Radiology and Nuclear Medicine], pp 184-188 (2005), and H. Schomberg et al in "Cone-beam CT using a C-arm system as front end and a spherical spiral as source trajectory", published in Proceedings Volume 7258, Medical Imaging 2009: Physics of Medical Imaging, 72580F (2009), available online at https://doi.org/10.1117/12.811545.

The so produced tomographic image *m* may then be displayed alongside, or instead of, some of the projection imagery or projection imagery earlier acquired (other than used for the reconstruction). The tomographic image *m* may be stored or otherwise processed, such as by a data consumer, such as an automated diagnostic system, possibly machine learning based, or for other purposes, as the case may be.

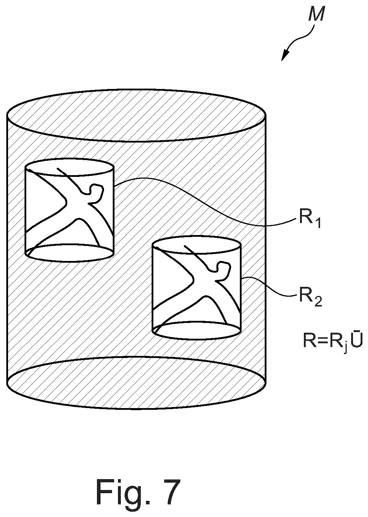

Reference is now made to Fig. 7 which shows an extension of the proposed method in that the region of interest may not necessarily be topologically connected but may instead be defined by unconnected "islets" *R1, R2* specified as specification as *s=(s1, s2)* as shown in image domain *M* in Fig. 7. For such disconnected, disjoint, region of interest *R* = ∪̅*ᵢ Rᵢ*, discretely spatially distributed into portions, the specification can be made separately for each islet *Ri* (by way of example, in the Figure only two such islets are shown, but there may be more than two).

For such embodiments, the above described multi-leaf collimator may be preferred as this allows defining multi-apertures masks that comprise more than one opening, with one opening collimated around a single respective one of the islets *Sj.* If such leaf collimator, or other collimator capable of forming arbitrary shaped disconnected aperture windows, is not available, a more restricted asymmetric collimator may be used instead, as described above, using one or two or more sets of blades. In such case, a collimator envelope, such as a circle or sphere or any other, may be defined that includes all islets *Sj,* and this is then used for collimation. Other approaches dealing with disconnected regions of interest are also envisaged herein. It should be noted that for disconnected reconstructions, it may be the case that at least one of the islets or portions of the region of interest is off-center as described above, or all such islets are off center, as the case may be.

The components of the facilitator system FS may be implemented as one or more software modules, run on one or more general-purpose processing units PU such as a workstation associated with the imager IA, or on a server computer associated with a group of imagers.

Alternatively, some or all components of the facilitator system FS may be arranged in hardware such as a suitably programmed microcontroller or microprocessor, such an FPGA (field-programmable-gate-array) or as a hardwired IC chip, an application specific integrated circuitry (ASIC), integrated into the imaging system IA. In a further embodiment still, the facilitator system FS may be implemented in both, partly in software and partly in hardware.

The different components of the facilitator system FS may be implemented on a single data processing system. Alternatively, some or more components are implemented on different processing system, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it, which computer program element is described herein.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs (set in brackets "()" in the claims) should not be construed as limiting the scope.

## Claims

1. System (FS) for facilitating imaging by a tomographic imaging apparatus (IA) having a collimator (COL) capable of asymmetric operation, comprising:-
an input interface (IN) for receiving, as input data, a specification (*s*) of a region of interest, ROI, (R) in 3D image domain (*M*) to be imaged for by the tomographic imaging apparatus (IA), the ROI (R) being off-center with respect to an iso-center of the imaging apparatus; and
a controller (CL) operable during, or in between, acquisitions by imaging apparatus (IA) of projection frames (*λj*) for tomographic reconstruction into a tomographic image *(m)* of the ROI (R), to cause dynamically adjusting, per frame, an aperture (*A, Aj*) of the collimator (COL) in respect of the so specified ROI.

2. System of claim 1, wherein the collimator adjustment includes repeatedly adjusting at least one blade (B) of collimator (COL) so as to define the per-frame aperture (*A, Aj*).

3. System of any one of the preceding claims, wherein the collimator (COL) is capable of asymmetric blade control.

4. System of any one of the preceding claims, wherein the controller (CL) is so operable based on a set of per frame collimation parameters (*a*) corresponding to the respective apertures, the set of said per frame collimation parameters determined by a collimation parameter determiner (AD) based on the specification (*s*).

5. System of any one of the preceding claims, wherein the iso-center is outside the specified ROI (R).

6. System of any one of the preceding claims, wherein the said specification is providable based on a scout image (*m*)* of at least a part of the imaging domain including the ROI (R).

7. System of any one of the preceding claims, wherein the system is capable of supporting a user interface, UI, (USI) configurable to allow user providing the specification.

8. System of any one of the preceding claims, wherein the ROI (R) comprises two or more disjoint portions (R1, R2).

9. System of any one of the preceding claims, wherein the said specification (s) is providable by an at least semi-automated specification provider (SP).

10. System of claim 9, wherein the said at least semi-automated specification provider (SP) includes a segmentor (SEG) capable of providing said specification in a segmentation operation.

11. System of any one of the preceding claims, wherein the tomographic image *(m)* is reconstructable by a reconstructor (RECON) capable of applying a tomographic reconstruction algorithm to the acquired projection frames (*λj*).

12. Imaging arrangement (IAR), comprising the system of any one of the preceding claims, and further comprising any one or more of: the imaging apparatus (IA), the UI, the collimator (COL), the specification provider (SP), the reconstructor (RECON), a memory (MEM) on which there is stored at least one of the scout image (*m**), the collimation parameters, and/or the said specification (s).

13. Computer-implemented method for facilitating tomographic imaging, comprising:-
receiving (S530), as input data, a specification (s) of a region of interest, ROI, (R) in 3D image domain (M) to be imaged for by the tomographic imaging apparatus (IA), the ROI (R) being off-center with respect to an iso-center of the imaging apparatus; and
during, or in between, acquisitions by imaging apparatus (IA) of projection frames (λj) for tomographic reconstruction into a tomographic image (*m*) of the ROI (R), dynamically adjusting (S620), per frame, an aperture (A, Aj) of the collimator (COL) in respect of the so specified ROI.

14. A computer program element, which, when being executed by at least one processing unit, is adapted to cause the processing unit to perform the method as per claim 13.

15. At least one computer readable medium having stored thereon the program element of claim 14.
